# EUROPEAN PATENT APPLICATION

(11) **EP 4 481 398 A1**
(43) Date of publication of application: **25.12.2024**
(21) Application number: 23756469.5
(22) Date of filing: 17.02.2023
(51) Int. Cl.: G01N 35/08, B81B 1/00, B81C 1/00, G01N 37/00

(54) **MICROCHIP FOR LIQUID SAMPLE ANALYSIS AND PRODUCTION METHOD THEREFOR**

(30) Priority: 18.02.2022 JP 2022024016
(71) Applicant: Fujimori Kogyo Co., Ltd., Tokyo 112-0002 (JP)
(72) Inventor: ABE, Masato, Tokyo 112-0002 (JP); HOSOKAWA, Kazuya, Tokyo 112-0002 (JP)
(74) Representative: Jones, Nicholas Andrew
(86) International application number: PCT/JP2023/005687
(87) International publication number: WO 2023/157945

(57) **Abstract**

A microchip for analyzing a liquid sample, internally including a flow path, includes: a substrate including a bonding surface provided with a groove that forms the flow path, a storage portion that stores a liquid in a portion of the groove, and a wall that blocks flow of the liquid from the storage portion to the flow path; a film that forms the flow path by attaching the film to the bonding surface of the substrate and covering the groove; and an adhesive portion that is formed by an adhesive agent for adhesively bonding the bonding surface of the substrate and the film to each other, and an adhesive agent that is present between an end surface of the wall and the film, and allows the liquid to be passed by peeling the end surface of the wall and the film by pressurization to the liquid.

## Description

### TECHNICAL FIELD

The present invention relates to a microchip for analyzing a liquid sample and a method of producing the microchip.

### BACKGROUND ART

It is known that a liquid sample such as blood is introduced into a flow path in a microchip and reacted with an antibody or the like in a reaction field provided in the middle of the flow path to analyze a component in the liquid sample. For preparation of such a microchip, a method of bonding a substrate on whose surface a groove serving as a flow path is formed with a film or a substrate using an adhesive agent is known.

### PRIOR ART REFERENCES

### PATENT DOCUMENTS

Patent Literature 1 JP2009-168667 A

### SUMMARY OF INVENTION

### PROBLEMS TO BE SOLVED BY THE INVENTION

In the case of using a plurality of liquids such as liquid samples, reagents, and washing liquids, the liquids are manually introduced into a flow path using a micropipette or the like. Therefore, operations become laborious, and a human error may occur. In contrast, for example, Patent Literature 1 discloses a microchip in which a reagent to be allowed to react with a liquid sample is stored in a recess in advance and sealed with a film. However, passage of a plurality of liquids into a flow path from sealed states requires such an effort that each film is removed. Therefore, a method of more inexpensively and more easily producing a microchip in which a liquid such as a reagent that is internally stored can be passed into a flow path in a simple operation has been desired.

An object of the present invention is to provide: a microchip in which a liquid that is internally stored can be easily passed into a flow path; and a method of producing the microchip.

### MEANS FOR SOLVING THE PROBLEM

In order to solve the above-described problem, the present inventors carried out an intensive study. As a result, it was found that a microchip can be easily produced by preparing a substrate including a bonding surface provided with a groove that forms a flow path, a storage portion that stores a liquid in a portion of the groove, and a wall that blocks the flow of the liquid from the storage portion to the flow path, preparing a film that forms the flow path by attaching the film to the bonding surface of the substrate and covering the groove, and applying an adhesive agent to a region, excluding the groove and the storage portion, of the bonding surface to adhesively bond the substrate and the film to each other, and the obtained microchip allows an end surface of the wall and the film that are adhesively bonded to each other to be peeled by pressurization to enable the liquid that is internally stored to be easily passed into the flow path.

In other words, a first aspect of the present invention is a microchip for analyzing a liquid sample, internally including a flow path, the microchip including: a substrate including a bonding surface provided with a groove that forms the flow path, a storage portion that stores a liquid in a portion of the groove, and a wall that blocks flow of the liquid from the storage portion to the flow path; a film that forms the flow path by attaching the film to the bonding surface of the substrate and covering the groove; and an adhesive portion that is formed by an adhesive agent for adhesively bonding the bonding surface of the substrate and the film to each other by applying the adhesive agent to a region, excluding the groove and the storage portion, of the bonding surface, and an adhesive agent that is present between an end surface of the wall and the film, and allows the liquid to be passed by peeling the end surface of the wall and the film by pressurization to the liquid.

A second aspect of the present invention is a method of producing a microchip for analyzing a liquid sample, internally including a flow path, the method including: a step of preparing a substrate including a bonding surface provided with a groove that forms a flow path, a storage portion that stores a liquid in a portion of the groove, and a wall that blocks the flow of the liquid from the storage portion to the flow path; a step of preparing a film that forms the flow path by attaching the film to the bonding surface of the substrate and covering the groove; a step of applying an adhesive agent to a region, excluding the groove and the storage portion, of the bonding surface, the adhesive agent adhesively bonding the bonding surface of the substrate and the film to each other and allowing the liquid to be passed by peeling an end surface of the wall and the film by pressurization to the liquid; and a step of attaching the film to the bonding surface of the substrate.

### EFFECTS OF THE INVENTION

According to the present invention, a microchip in which a liquid that is internally stored can be easily passed into a flow path, and a method of producing the microchip can be provided.

### BRIEF DESCRIPTION OF THE DRAWINGS

Fig. 1 is a diagram illustrating the substrate of a microchip before application of an adhesive agent.
Fig. 2 is a diagram illustrating the substrate of the microchip after the application of the adhesive agent.
Fig. 3 is a final drawing of the microchip in which a film is attached to the substrate.
Fig. 4 is a diagram illustrating a microchip in a state in which an adhesive agent is peeled and a liquid is passed.
Fig. 5 is a diagram illustrating a portion of a cross section, taken along the line A-A, of the microchip.
Fig. 6 is a diagram illustrating a portion of a cross section, taken along the line B-B, of the microchip in which a liquid is passed.
Fig. 7 is a diagram for explaining an example of application of the liquid-sealed structure of a microchip.
Fig. 8 is a diagram representing the structure of the substrate of a microchip in Example 1 or 2.
Fig. 9 is a diagram representing a portion, to which an adhesive agent is applied, of the substrate of a microchip in Example 3.
Fig. 10 is a diagram illustrating a portion of a cross section, taken along the line C-C, of the microchip in Example 3.

### EMBODIMENTS FOR CARRYING OUT THE INVENTION

The present invention provides: a microchip in which a liquid in a storage portion is sealed by a wall that blocks a flow path and the storage portion formed in a portion of the flow path and an adhesive agent applied to an end surface of the wall, and the end surface of the wall and the film are peeled by pressurization to the liquid, whereby the liquid can be passed; and a method of producing the microchip.

The liquid is, for example, a liquid sample, a reactant that is allowed to react with a liquid sample, a washing liquid, or the like. Such a liquid sample is not particularly restricted as long as the sample can be passed into the microchip, and examples thereof include a liquid sample obtained from a living body, such as blood or urine, or a diluted liquid thereof, an extract from a living body, such as a plant or animal, naturally occurring water, such as river, ocean, or rainfall, washing liquid, and waste liquid. A component in the sample is not particularly restricted, and examples thereof include a protein, a nucleic acid, a low molecular weight compound, and a sugar.

A storage portion which is a region in which the liquid is stored is formed in a portion of the flow path in the microchip, and the liquid is stored in the storage portion in advance. The wall disposed between the storage portion and the flow path blocks flow of the liquid from the storage portion into the flow path. The adhesive agent applied to the end surface of the wall seals the liquid so that the liquid is prevented from flowing into the flow path. The end surface of the wall and the film are peeled to pass the liquid from the storage portion into the flow path by pressurizing the liquid in the storage portion.

The microchip of the present invention includes a liquid-sealed structure in which a liquid is sealed with the wall and the adhesive agent as described above, and therefore enables a liquid such as a liquid sample, a reactant, or a washing liquid to be introduced into the flow path in a simple operation. Therefore, a laborious operation in which a liquid is manually introduced into a flow path by using a micropipette or the like is reduced.

The microchip of the present invention includes: a substrate including a bonding surface provided with a groove that forms a flow path, a storage portion that stores a liquid in a portion of the groove, and a wall that blocks flow of the liquid from the storage portion to the flow path; a film that forms the flow path by attaching the film to the bonding surface of the substrate with an adhesive agent applied to a region, excluding the groove and the storage portion, of the bonding surface, and covering the groove; and an adhesive portion that is formed by the adhesive agent that allows the liquid to be passed by adhesively bonding the substrate and the film and peeling an end surface of the wall and the film by pressurization to the liquid.

Hereinafter, the microchip for analyzing a liquid sample of the present invention and the method of producing the microchip will be described with reference to the drawings. However, the following is only an example, and the manufacturing method of the present invention and a microchip obtained by the method are not limited to the following aspects.

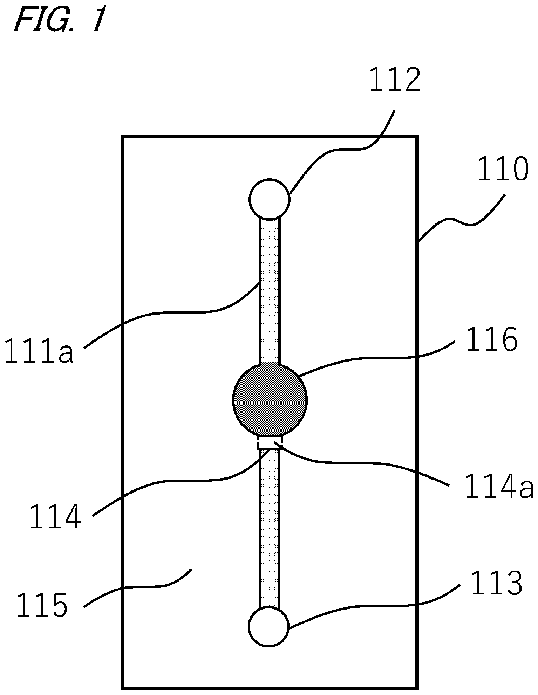

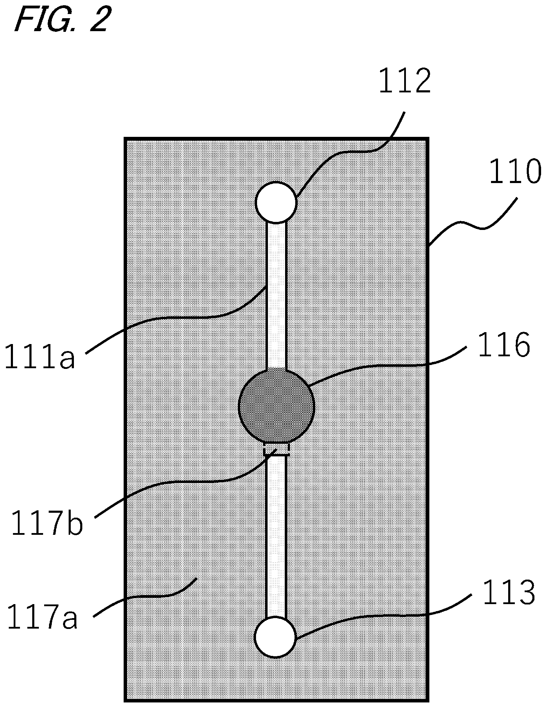

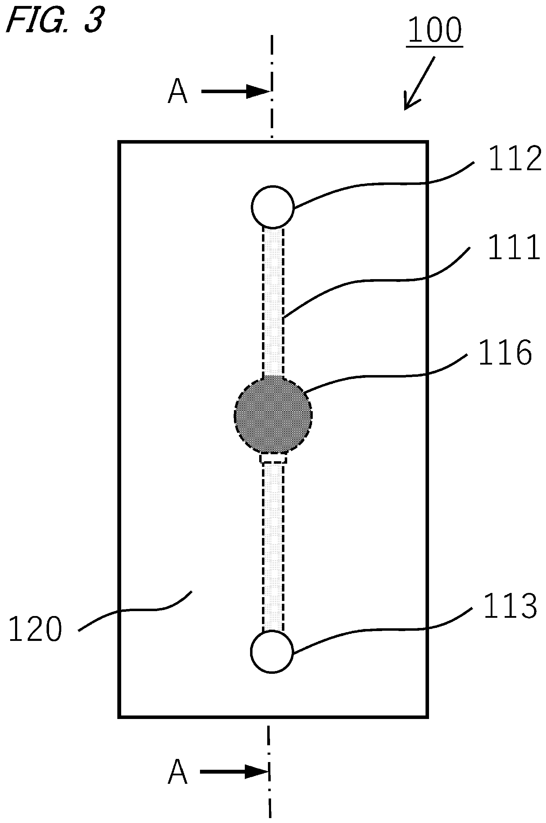

Figs. 1 to 3 are conceptual diagrams illustrating examples of a form in a process of producing a microchip 100. Fig. 1 is a diagram illustrating the substrate 110 of the microchip 100 before application of an adhesive agent. Fig. 2 is a diagram illustrating the substrate 110 of the microchip 100 after the application of the adhesive agent. Fig. 3 is a final drawing of the microchip 100 in which a film 120 is attached to the substrate 110.

Fig. 1 illustrates a plan view of the substrate 110 on the surface of which a groove 111a that forms the flow path 111 of the microchip 100 is carved. A portion of the groove 11 1a is provided with a depression serving as a storage portion 116 which is a region in which a liquid is stored. A wall 114 that blocks flow of the liquid is disposed between the storage portion 116 and the groove 111a in a side in which the liquid flows. A surface, facing the attached film 120, of the wall 114 is also referred to as an end surface 114a. A surface on which the groove 111a is carved is a surface that is attached to the film 120, and is also referred to as a bonding surface 115.

A through-hole serving as an inlet 112 for pressurizing a liquid is disposed in one end of the groove 111a, closer to the storage portion 116 than the wall 114, and a through-hole serving as an outlet 113 (exhaust port) for allowing a liquid or air to flow is disposed in the other end. A pressure is applied to a liquid stored in the storage portion 116 by using, for example, a micro syringe, a pump, or the like. Press with a finger with which the inlet 112 is blocked also enables pressurization to a liquid. The inlet 112 can be sealed using a seal or the like until just before use in order to prevent evaporation of the liquid stored in the storage portion 116.

Two or more flow paths 111 may be disposed. The shape of the flow path 111 may be any shape, and may be straight or curved. The flow path 111 may include a branch. In such a case, the flow path may include two or more inlets 112, storage portions 116, walls 114, and/or outlets 113.

For example, a liquid sample is stored in a first storage portion disposed in a first flow path, and a reaction substrate liquid (reactant) is stored in a second storage portion disposed in a second flow path. By pressurizing the liquid sample and the reaction substrate liquid from inlets corresponding thereto, the end surface of the wall and the film are peeled, and the liquid sample and the reaction substrate liquid are passed into the flow path over the storage portion. The first flow path and the second flow path are confluent at a point over the first storage portion and the second storage portion. Disposition of a reaction field in a confluent region causes the liquid sample to react with the reaction substrate liquid. In such a case, the outlet may be disposed at the downstream end of the confluent flow path.

It is also acceptable to store a washing liquid in a third storage portion disposed in a third flow path and to pass the washing liquid into the flow path of the microchip 100 after reaction between a liquid sample and a reaction substrate liquid. For example, the third flow path is formed to be confluent in an upstream side of the first storage portion and the second storage portion, and the washing liquid is passed into the first flow path and the second flow path by the pressurization from the inlet. In pressurization from any one flow path, it is necessary to prevent backward flow of a liquid to another flow path, in a case in which two or more flow paths exist. In such a case, a through-hole in an end of a flow path other than the flow path from which the pressurization is performed may be sealed with a seal or the like.

The inlet 112 and the outlet 113 may be disposed on either side of the substrate 110 or the film 120. For example, the groove 111a serving as the flow path 111 may be disposed on the substrate 110, and the film 120 provided with through-holes at positions overlapping both ends of the groove 111a may be prepared and attached to the substrate 110. One of the through-holes serving as the inlet 112 and the outlet 113 may be disposed on the substrate 110, and the other may be disposed on the film 120.

The cross-sectional shape of the groove 111a serving as the flow path 111 may be any shape, such as concave, U-shaped, or V-shaped. The depth of the groove 111a serving as the flow path 111 is preferably from 10 to 500 µm, and the width of the groove is preferably from 10 µm to 3 mm. The length of a portion corresponding to the flow path 111 including the thickness of the wall 114 (the longitudinal length of the flow path 111) is, for example, from 3 mm to 5 cm. The width of the groove 111a may be constant or may vary. The depth of the groove 111a may also be constant, but may vary.

The width of the groove 111a (the width of the flow path 111) may be allowed to be less than the width of the wall 114. The flow rate of a liquid passing through a gap between the film 120 and the end surface 114a of the wall 114 is increased to facilitate flow of the liquid by allowing the width of the wall 114 to be more than the width of the flow path 111.

A depression serving as the storage portion 116 may be of any size as long as the depression is large enough to store a desired amount of liquid, and the shape of the depression is also not restricted. For example, the depression may be cylindrical or prismatic, and by increasing the area and depth, a larger amount of liquid can be stored. The area of the depression is, for example, from 0.1 to 50 mm², and in the case of the storage portion 116 having a circular shape, the diameter thereof is, for example, from 0.2 to 6 mm. However, the area may vary with the depth of the groove, and the shape of the depression may be, for example, mortar-shaped. The depth of a depression is preferably deeper than the depth of the groove serving as the flow path, and is, for example, from 20 µm to 3 mm.

The wall 114 is disposed to block flow of a liquid stored in the storage portion 116 into the flow path 111. The gap between the end surface 114a of the wall 114 and the film 120 covering the end surface 114a of the wall 114 is sealed with an adhesive agent 117b that is applied to the end surface 114a. The liquid in the storage portion 116 does not flow into the flow path 111 in the state of being sealed with the adhesive agent 117b.

The thickness of the wall 114 (the longitudinal length of the flow path 111) may be a thickness allowing a pressurized liquid to result in peeling between the film 120 and the end surface 114a of wall 114 and to pass from the storage portion 116 toward the flow path 111. The width of the wall 114 may be constant or may vary. The thickness of the wall 114 is preferably set at 4 mm or less, more preferably 2 mm or less, in order to enhance peelability and sufficiently secure the flow rate of liquid. The lower limit of the thickness of the wall 114 is not particularly limited as long as the lower limit is in a range in which an adhesive agent can be applied to the end surface 114a and a liquid in the storage portion can be sealed. The wall surface of the wall 114 may be a sloped surface allowing the thickness of the wall 114 to decrease with approaching the end surface 114a. By allowing the wall surface of the wall 114 to have such a structure that the wall surface is a sloped surface or stepwise and the depth of the storage portion 116 decreases with approaching the wall 114, flow of a liquid between the end surface 114a and the film 120 is facilitated, and peeling of the adhesive agent 117b from the end surface 114a or the film 120 is facilitated. The wall 114 may be formed so that the height of the wall 114 is generally equal to the height of the bonding surface 115 or less than the height of the bonding surface 115.

The peeling between the film 120 and the end surface 114a of the wall 114 may be cohesion failure of the layer of the adhesive agent 117b, may be interfacial peeling between the end surface 114a of the wall 114 and the layer of the adhesive agent 117b, or may be interfacial peeling between the film 120 and the layer of the adhesive agent 117b.

The size of a through-hole serving as the inlet 112 may be any size that allows pressurization of a liquid in the storage portion 116 using a micro syringe, a pump connected by an adaptor, or the like. The size of the through-hole serving as the inlet 112 is, for example, a diameter of 0.2 to 3 mm. The size of a through-hole serving as the outlet 113 is not particularly restricted, as long as the through-hole is large enough to function as an outlet for a liquid sample. The size of the through-hole serving as the outlet 113 is, for example, a diameter of 0.2 to 3 mm.

Metal, glass, plastic, silicone, or the like can be used as the material of the microchip 100, and from the viewpoint of detecting a reaction by luminescence, coloration, or visual inspection, a transparent material is preferable, and a transparent plastic is more preferable. Examples of the material of the microchip 100 include polyethylene, polypropylene, polystyrene, polymethyl methacrylate, cycloolefin polymer, cycloolefin copolymer, polyphenylene oxide, polyethylene terephthalate, polyethylene naphthalate, polycarbonate, polyamide, polyimide, a phenol resin, an epoxy resin, a polyvinylidene chloride, a polyvinyl chloride, an acrylonitrile-butadiene-styrene (ABS) resin, and a poly(2-methoxyethyl acrylate) (PMEA) resin.

The groove 111a or a through-hole disposed in the substrate 110 of the microchip 100 can be engraved with a blade or a laser beam, and when the material of the microchip 100 is plastic, the groove or through-hole can also be formed by injection molding. Formation by injection molding is preferable since the microchip 100 of consistent quality can be produced efficiently.

The end surface 114a of the wall to which the adhesive agent 117b that is peeled by pressurization to a liquid is applied, and a portion of the film 120, covering the end surface 114a, may be subjected to surface treatment that reduces the adhesive strength of the adhesive agent 117b.

The surface treatment (hydrophobization treatment) that reduces the adhesive strength is preferably fluorine membrane coating by application or plasma treatment of a hydrophobization reagent. Examples of the hydrophobization reagent include FLUOROSURF FS-1610C-4.0 (FluoroTechnology Co., LTD.), water-repellent oil-repellent fluorine coating agent GF03 (GAST JAPAN Co., Ltd.), Guard Surf AZ-6000 (Harves Co., Ltd.), and fluorine coating agent INT series (Noda Screen Co., Ltd.). Examples of specific conditions include a condition where the water contact angle of the end surface of the wall is, for example, 110° or more.

The end surface 114a of the wall to which the adhesive agent 117b is peeled by pressurization to a liquid, and the portion of the film 120, covering the end surface 114a, may be subjected to surface treatment that reduces adhesive strength, and only a region other than the end surface 114a of the wall to which the adhesive agent 117b is applied, and the portion of film 120, covering the end surface 114a, may be subjected to surface treatment that improves adhesive strength. The end surface 114a of the wall, to which the adhesive agent 117b is applied, and the portion of the film 120, covering the end surface 114a, can be untreated.

The surface treatment that improves adhesive strength is preferably corona treatment, plasma treatment, or excimer treatment. A gaseous species in the case of using plasma treatment is not restricted, argon, oxygen, or nitrogen is preferred, and air plasma treatment under vacuum is particularly preferred. Examples of specific conditions include a condition where the water contact angle of the end surface of the wall is, for example, 30° or less.

Portions of the film 120 and/or the substrate 110 may be hydrophilized to control the direction of flow of a liquid. For example, the groove 111a serving as the flow path 111 of the substrate 110, the portion of film 120, covering the flow path 111, both the wall surfaces of the wall 114, and the like are hydrophilized. A depression serving as the storage portion 116 and a portion of the film 120, covering the storage portion 116, may be subjected to hydrophilization treatment.

The hydrophilization treatment is preferably performed by applying a hydrophilization reagent or a plasma treatment. Examples of the hydrophilization reagent include a nonionic surfactant such as S-1570 (sucrose fatty acid esters: MITSUBISHI-CHEMICAL FOODS CORPORATION), LWA-1570 (sucrose laurate: MITSUBISHI-CHEMICAL FOODS CORPORATION), POEM DL-100 (diglycerin monolaurate: RIKEN VITAMIN Co., Ltd.), or RIKEMAL A (sucrose fatty acid esters: RIKEN VITAMIN Co., Ltd.), CeraAqua NS235-N1 (SHIMA TRADING CO., LTD.), Aminoion (NIPPON NYUKAZAI CO., LTD.), LAMBIC-771W (OSAKA ORGANIC CHEMICAL INDUSTRY LTD.), LAMBIC-1000W (OSAKA ORGANIC CHEMICAL INDUSTRY LTD.), SPRA-101 (TOKYO OHKA KOGYO CO., LTD.), and SPRA-202 (TOKYO OHKA KOGYO CO., LTD.). Examples of specific conditions include a condition where the water contact angle of a substrate surface is, for example, 55° or less.

The material of the film 120 is preferably a transparent plastic, and the materials described above are exemplified, and a polyethylene terephthalate (PET) resin, a cycloolefin polymer (COP) resin, a cycloolefin copolymer (COC) resin, a polystyrene (PS) resin, a polycarbonate (PC) resin, or a polymethyl methacrylate (PMMA) resin is more preferable. The thickness of the film 120 is, for example, preferably from 50 to 200 µm, and more preferably from 100 to 200 µm.

### <Step of Applying Adhesive Agent and/or Gluing Agent>

In order to attach the film 120 onto the substrate 110, an adhesive agent and/or a gluing agent are used. Examples of the adhesive agent include a (meth)acrylic resin-based adhesive, a natural rubber adhesive, a urethane resin-based adhesive, an ethylene-vinyl acetate resin emulsion adhesive, an ethylene-vinyl acetate resin-based adhesive, an epoxy resin-based adhesive, a vinyl chloride resin solvent-based adhesive, a chloroprene rubber-based adhesive, a cyanoacrylate-based adhesive, a silicone-based adhesive, a styrene-butadiene rubber solvent-based adhesive, a nitrile rubber-based adhesive, a nitrocellulose-based adhesive, a phenolic resin-based adhesive, a modified silicone-based adhesive, a polyester-based adhesive, a polyamide-based adhesive, a polyimide-based adhesive, an olefin resin-based adhesive, a polyvinyl acetate resin emulsion-based adhesive, a polystyrene resin solvent-based adhesive, a polyvinyl alcohol-based adhesive, a polyvinyl pyrrolidone resin-based adhesive, a polyvinyl butyral-based adhesive, a polybenzimidazole adhesive, a polymethacrylate resin solvent-based adhesive, a melamine resin-based adhesive, a urea resin-based adhesive, and a resorcinol-based adhesive. One or more adhesive agents can be used singly, or two or more kinds thereof can be used in mixture.

In order to attach the film 120 onto the substrate 110, a gluing agent which is one kind of adhesive agents may be used. Examples of the gluing agent include a rubber-based adhesive, a (meth)acrylic adhesive, a silicone-based adhesive, a urethane-based adhesive, a vinyl alkyl ether-based adhesive, a polyvinyl alcohol-based adhesive, a polyvinyl pyrrolidone-based adhesive, a polyacrylamide-based adhesive, and a cellulose-based adhesive. Such gluing agents may be used singly, or two or more kinds thereof may be used in mixture.

The adhesive agent or gluing agent is preferably light-curing (either radical reactive or cationic polymerization), and more preferably ultraviolet-curing (UV-curing). With a UV curing adhesive agent or gluing agent, after an application process, irradiation with UV light quickly initiates a curing reaction, allowing bonding to take place. For the UV curing adhesive agent, for example, an acrylic UV curing adhesive agent such as UVX-8204 (manufactured by Denka Company Limited.), UVX-8400 (manufactured by Denka Company Limited.), SX-UV 100A (manufactured by CEMEDINE CO., LTD.), SX-UV200 (manufactured by CEMEDINE CO., LTD.), BBX-UV300 (manufactured by CEMEDINE CO., LTD.), U-1340 (Chemitech Inc.), U-1455B (Chemitech Inc.), U-1558B (Chemitech Inc.), Aronix UV-3000 (TOAGOSEI CO., LTD.), TB3094 (ThreeBond Co., Ltd.), or Hitaroid 7975D (Hitachi Chemical Company, Ltd.) is more preferable. For the UV curing gluing agent, an acrylic UV curing gluing agent such as UV-3630ID80 (Mitsubishi Chemical Corporation), UX-3204 (Nippon Kayaku Co., Ltd.), or FINETAC RX-104 (DIC Corporation) is more preferable. An acrylic UV curing adhesive agent and gluing agent can exhibit favorable adhesion to a wide range of plastic materials and achieve rapid strength development after UV irradiation. The viscosity of an adhesive agent and a gluing agent used for attaching the film 120 onto the substrate 110 is preferably, for example, from 2,000 to 31,000 mPa·s.

A step of preparing the substrate 110 and applying an adhesive agent 117a and the adhesive agent 117b to the bonding surface 115 by screen printing is described with reference to Fig. 2. The adhesive agent 117a and the adhesive agent 117b are also generically referred to as an adhesive agent 117. In a case in which the film 120 is attached to the substrate 110 with a gluing agent which is one kind of adhesive agents, the gluing agent may be applied to the substrate 110 in a manner similar to that in the case of the adhesive agent 117. Fig. 2 is a diagram illustrating the substrate 110 of the microchip 100 after the application of the adhesive agent.

A description is given below for a case using an adhesive agent. However, the following also applies to a case where a gluing agent is used instead of the adhesive agent. Thus, the term "adhesive agent" may be replaced by the term "adhesive agent and/or gluing agent".

The adhesive agent 117a and the adhesive agent 117b may be simultaneously applied, or may be individually applied. The adhesive agent 117a is applied to a region excluding the groove 111a disposed in the bonding surface 115 of the substrate 110, the storage portion 116, and the end surface 114a of the wall 114. The adhesive agent 117b is applied to the end surface 114a of the wall 114. The adhesive agent 117a and the adhesive agent 117b may be the same kind of an adhesive agent, or may be different kinds of adhesive agents.

The adhesive agent 117b between the film 120 and the end surface 114a of the wall 114 may be applied to a downstream portion of the flow path of a liquid in the end surface 114a of the wall 114. In other words, the adhesive agent 117b may be applied to an almost half of the downstream side of the end surface 114a of wall 114 with respect to the direction of the flow path. In such a case, pressurization from a through-hole in an upstream side to which the adhesive agent 117b of the end surface 114a is not applied facilitates flow of a liquid between the end surface 114a and the film 120, and peeling between the adhesive agent 117b and the end surface 114a or the film 120.

For more accurately applying the adhesive agent 117a and the adhesive agent 117b to corresponding application regions, the adhesive agent 117 is preferably applied by printing technique, and particularly preferably applied by screen printing in a case in which the adhesive agent 117a and the adhesive agent 117b are the same kind of an adhesive agent. By using screen printing, even when a screen printing plate in a region corresponding to the entire surface of the substrate 110 is filled with the adhesive agent 117, the adhesive agent 117 is transferred to an application region that comes into contact with the screen printing plate, but the adhesive agent 117 is not transferred to the groove 111a and a depression serving as the storage portion 116 that do not come into contact with the screen printing plate. Thus, the adhesive agent 117a and the adhesive agent 117b can be favorably applied to corresponding application regions.

The film thickness of the adhesive agent 117 that is applied to the substrate 110 is preferably from 5 to 15 µm. For controlling the film thickness of the adhesive agent 117, the mesh count per inch of screen is preferably, for example, from 500 to 730. The opening ratio of the mesh is preferably, for example, from 39 to 47%. The thickness of a mesh is preferably, for example, from 15 to 28 µm. With this, the film thickness of the applied adhesive agent 117 is preferably from 5 to 15 µm.

As other methods of applying the adhesive agent 117 to the substrate 110, inkjet printing, gravure printing, or a dispenser can be used to precisely apply the adhesive agent 117a and the adhesive agent 117b to corresponding application regions. In these application techniques, when the adhesive agent 117 is discharged against the groove 111a, the adhesive agent 117 applied inside the groove 111a changes the shape of the flow path 111. Therefore, printing treatment and the operation of the dispenser may be controlled by a program so that the adhesive agent 117a and the adhesive agent 117b are applied to corresponding application regions by capturing an image of the position of the groove 111a of the substrate 110 or fixing a printing stage and the position of the substrate 110.

The adhesive agent 117a may be applied after hydrophilization treatment of a region excluding the groove 111a disposed in the bonding surface 115 of the substrate 110, the storage portion 116, and the end surface 114a of the wall 114. The hydrophilization treatment is preferably plasma treatment or corona treatment. The substrate 110 and the film 120 can be favorably attached to each other by performing the hydrophilization treatment so that the substrate 110 does not repel the adhesive agent 117a, and the adhesive agent 117a spreads on the substrate 110 and does not flow into the groove 111a.

### <Attachment Step>

A step of preparing the film 120 and attaching the film 120 to the substrate 110 is described with reference to Fig. 3. Fig. 3 is a final drawing of the microchip in which the film 120 is attached to the substrate 110. Fig. 3 illustrates a plan view of the microchip 100 obtained by attaching the film 120 and the bonding surface 115, on which the groove 111a of the substrate 110 and the storage portion 116 are engraved, to each other. The flow path 111 formed in the interior of the microchip 100 is indicated by the dashed lines.

The same material as the material of the substrate is preferably used for the film 120. As a result, peeling caused by a difference in shrinkage percentage or expansion percentage between a substrate and a film can be prevented in a case in which a temperature environment in which a produced microchip is stored is changed. The thickness of the film 120 is preferably 100 µm or less, and particularly preferably 50 µm or less, depending on a material. When the end surface 114a and the film 120 are peeled by pressurization, the peeling is facilitated by force in a direction from the interior of the flow path toward a surface of the film 120, in a case in which the thickness is 50 µm or less.

By layering the film 120 on the substrate 110 and attaching the film 120 and the substrate 110 to each other, the film 120 covers the upper portions of the groove 111a and a depression serving as the storage portion 116, the flow path 111, through which a liquid passes, and the storage portion 116 are formed. The adhesive agent 117a and the adhesive agent 117b are cured to form an adhesive portion by UV irradiation. The bonding between the substrate 110 and the film 120 and the formation of a structure in which liquid flow is blocked can be achieved in the same step by applying the adhesive agents (117a, 117b) to a predetermined application region by screen printing in such a manner, layering the film 120 on the substrate 110, and attaching the film 120 and the substrate 110 to each other.

By layering the film 120, a side, closer to the bonding surface 115, of a through-hole of the substrate 110 is sealed, and only a surface reverse to the bonding surface 115 serves as an inlet. This allows the through-hole of the substrate 110 to function as the inlet 112 and the outlet 113.

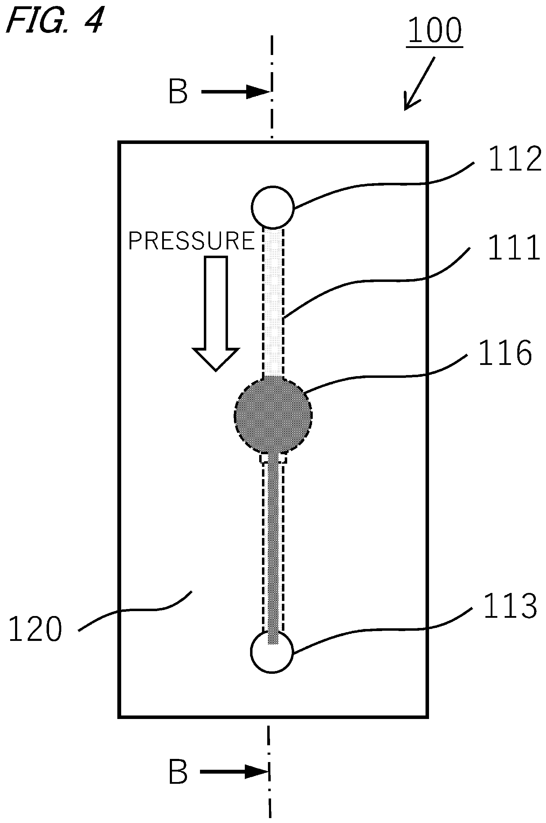

Passing of a liquid stored in the storage portion 116 is described with reference to Fig. 4. For example, in a case in which a washing liquid is stored in the storage portion 116, the film and the end surface 114a of the wall 114 are peeled to pass the washing liquid into the downstream side of the flow path 111 by pressurization from the inlet 112. The washing liquid washes the flow path 111 in the downstream side, the storage portion 116 for another liquid disposed in a portion of the flow path 111 in the downstream side, a reaction field, and the like, and is discharged from the outlet 113.

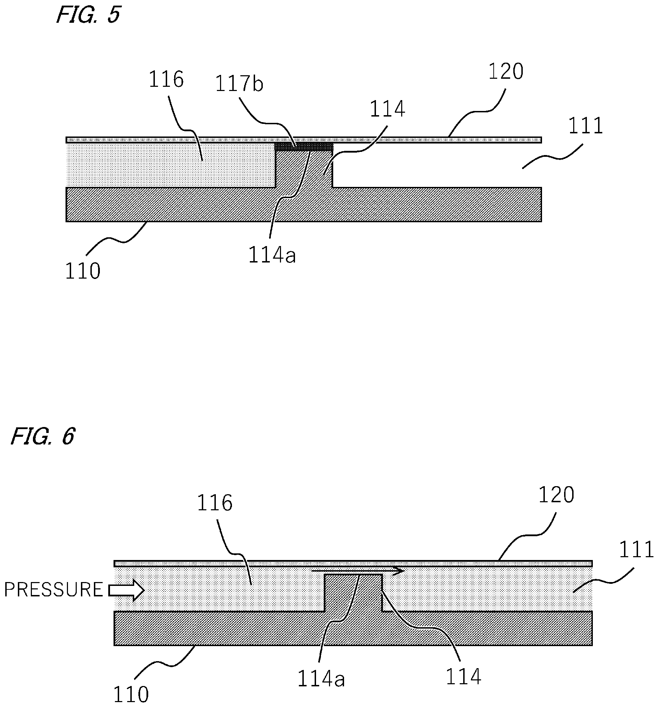

Passing of a liquid stored in the storage portion 116 into the flow path 111 is described with reference to Figs. 5 and 6. Fig. 5 is a diagram illustrating a portion of a cross section, taken along the line A-A, of the microchip 100 illustrated in Fig. 3. The film 120 is affixed to the substrate 110, and a gap between the film 120 and the end surface 114a of the wall 114 is filled with the adhesive agent 117b. The liquid stored in the storage portion 116 is sealed with the wall 114 and the adhesive agent 117b, and does not flow into the flow path 111.

Fig. 6 is a diagram illustrating a portion of a cross section, taken along the line B-B, of the microchip 100 illustrated in Fig. 4, in which the film 120 and the end surface 114a of the wall 114 are peeled, and a liquid is passed into the downstream side of the flow path 111. By pressurization from the inlet 112, the liquid in the storage portion 116 allows the end surface 114a and the film 120 to be peeled, and is passed into the downstream side of the flow path 111 through a gap between the end surface 114a and the film 120. The liquid stored in the storage portion 116 is pressurized using a micro syringe or a pump through the inlet 112, or pressurized by press with a finger.

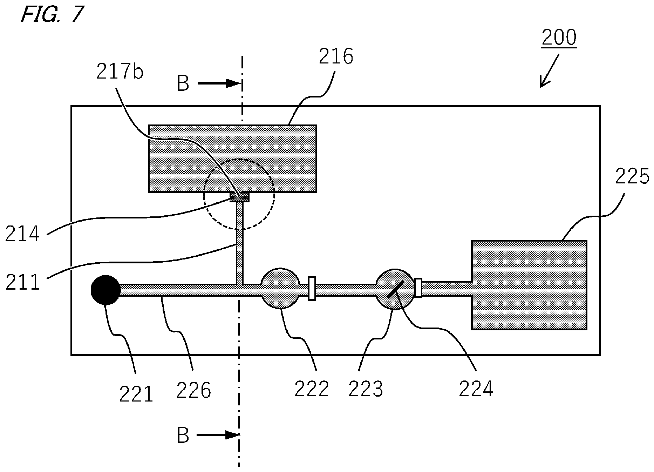

Fig. 7 is a diagram for explaining an example of application of the liquid-sealed structure of a microchip. The microchip 200 illustrated in Fig. 7 includes a washing liquid tank 216 (corresponding to the storage portion 116) storing a washing liquid. A wall 214 is disposed between the washing liquid tank 216 and a flow path 211. The washing liquid in the washing liquid tank 216 is sealed with the wall 214 and an adhesive agent 217b applied to an end surface of the wall 214. In a cross section taken along the line B-B, a portion (portion surrounded by the dotted line) including the wall 214 includes a cross section similar to a cross section in Fig. 5.

Moreover, the microchip 200 includes an inlet 221, a storing portion 222 for a reactant and the like, a reaction field 223, a waste liquid tank 225, and a flow path 226 that communicates between the inlet 221 and the waste liquid tank 225. A liquid sample is introduced from the inlet 221 into the flow path 226. The storing portion 222 stores a reactant such as a lyophilized antibody. In the reaction field 223, for example, a magnetic bead formed by immobilizing a secondary antibody as the reactant, and the like are stored, and a stirring bar 224 is placed. The stirring bar 224 promotes reaction by mixing the liquid sample flowing into the reaction field 223 with a magnetic bead formed by immobilizing a different kind of a reactant, together with the reactant stored in the storing portion 222.

The reactant can be any substance that reacts with a target (detection target) component in a liquid sample, and can be appropriately selected according to the type of a target substance. Examples of the reactivity of a reactant include a biological reaction and a chemical reaction, and examples of the biological reaction include a binding reaction. Examples of the reactant include a protein (including a peptide), a sugar, a nucleic acid, and a low molecular weight compound. Examples of the reactant include a substance such as an antibody that binds specifically to a target substance, an enzyme protein that uses a target substance as a matrix, and a blood coagulation factor such as a PT reagent. When the target substance is a nucleic acid, the reactant may be a nucleic acid probe or a polymerase (nucleic acid amplifying enzyme) that amplifies a nucleic acid. Two or more reactants may be used, and in the example of Fig. 7, different reactants are stored in the storing portion 222 and the reaction field 223, respectively.

A target substance in a sample can be measured by observing or detecting a reaction in the reaction field 223. Examples of the reaction include, but are not limited to, a chromogenic reaction, a luminescence reaction, an amplification reaction, and an aggregation reaction. The liquid sample subjected to the reaction flows into the downstream side of the flow path 226, and is stored in the waste liquid tank 225.

Hereinafter, the present invention will be specifically described with reference to Examples, but the present invention is not limited to the following aspects.

### EXAMPLE 1

### <Microchip Preparation 1>

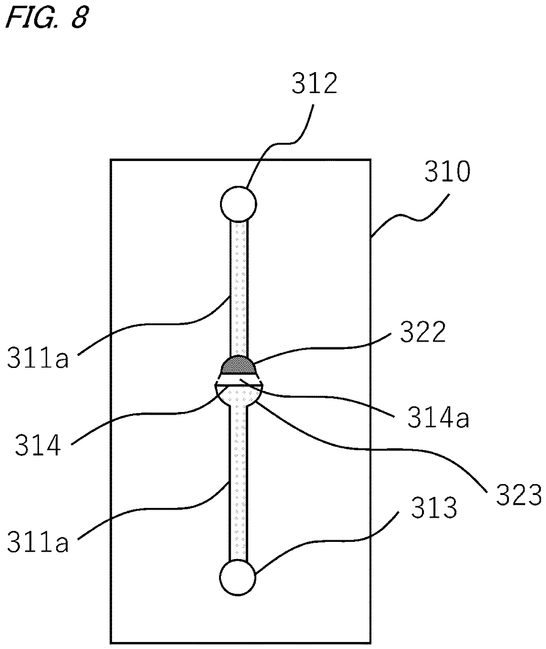

A substrate 310 (injection molded product manufactured by MCC Advanced Moldings Co., Ltd.: COP resin) (size of 59.4 × 26.2 mm, thickness of 3.0 mm) illustrated in Fig. 8 was prepared. In the substrate 310, a groove 311a forming a flow path has a length of 47.4 mm, a depth of 500 µm, and a width of 500 µm at an inlet, and a storage portion 322 having a semicircular shape formed by half-cutting a circle having a diameter of 2 mm, of which the center is a point of 37.55 mm in the direction from the inlet to an outlet is included. A side that received a liquid passed from the storage portion 322 was a storage portion 323 having a semicircular shape formed by half-cutting a circle having a diameter of 4 mm. A wall 314 having a width of 2 mm is included between flow paths in the side that received the liquid from the storage portion 322. In the substrate, a hole serving as an inlet 312 and an outlet 313 was a through-hole with a circular cross sectional shape with an inner diameter of 2 mm.

The groove 311a and the storage portion 322 having a semicircular shape formed by cutting a circle having a diameter of 2 mm were filled with distilled water by dipping the through-hole (inlet 312) into distilled water under reduced pressure.

An adhesive agent was used to attach the substrate 310 and the film to each other. The adhesive agent was UVX-8204, a solvent-free, radical reactive acrylic UV curing adhesive agent. The adhesive agent was applied to a surface provided with the groove 311a of the substrate 310 by screen printing. In a screen plate used, the mesh count was 640, the opening ratio was 39%, and the application thickness of the adhesive agent was about 7 µm.

The film used for bonding was ZF14-100 (manufactured by Zeon Corporation: COP) (thickness 100 µm), ZF14-050 (manufactured by Zeon Corporation: COP) (thickness 50 µm), or ZF14-023 (manufactured by Zeon Corporation: COP) (thickness 23 µm).

The adhesive agent applied to a surface of the substrate 310 was layered with the film and irradiated with ultraviolet light having a wavelength of 365 nm for 10 to 20 seconds using a UV-LED light source to initiate a curing reaction of the adhesive agent and bond the film onto the substrate 310.

### <Microchip Evaluation 1>

The inlet 312 was connected to a syringe pump for pressurization using a silicon tube and a connector produced by a bush made of an ABS resin. By assembling a pressure sensor to the syringe pump, a pressure at the moment when the adhesive agent on an end surface 314a of the wall 314 was peeled was recorded on the basis of a peak hold function. As a result of pressurization by the syringe pump, in the microchip produced by attaching the film ZF14-050 or ZF14-023, the adhesive agent on the end surface 314a of the wall 314 was peeled at 500 kPa, and the distilled water in the storage portion 322 flowed into the following flow path. In contrast, in the microchip produced by attaching ZF14-100, liquid leakage occurred at points except the wall 314 at 800 kPa.

This revealed that in the microchip produced by disposing the wall 314 between the storage portion 322 and the storage portion 323, applying the adhesive agent to the end surface 314a of the wall 314, and bonding the end surface 314a to the film, liquid was able to be passed at optional timing by pressurization from the storage portion 322.

A liquid can be passed at a lower pressure by reducing the width of the wall 314. Moreover, a similar effect is obtained by decreasing a depth to facilitate application of a pressure to the adhesive agent of the end surface 314a in an area nearer to the wall 314 closer to the storage portion 322.

### EXAMPLE 2

It was confirmed whether bonding strength was able to be improved by surface-treating a substrate 310 and a film, and peelability between a wall 314 and the film was able to be improved by allowing only an end surface 314a of the wall 314, intended to be peeled, to be untreated.

### <Microchip Preparation 2>

A substrate 310 similar to the substrate in Example 1 was used as a substrate used in production. ZF14-100 was used as a film.

A groove 311a and a storage portion 322 having a semicircular shape formed by cutting a circle having a diameter of 2 mm were filled with distilled water by dipping a through-hole (inlet 312) into distilled water under reduced pressure.

Surface treatment of the substrate 310 was performed using an atmospheric pressure plasma irradiation machine prior to application of an adhesive agent. In this case, a mask was produced on only an end surface 314a of a wall 314 with a Kapton tape, and the end surface 314a was prevented from being irradiated with plasma. The water contact angle of a surface-treated region was 30°, and the water contact angle of the end surface 314a was kept at 85° equivalent to that of an untreated COP surface.

Surface treatment of the film was performed using a corona irradiation machine prior to attachment. In this case, in the attachment, a mask was produced on only a region corresponding to the end surface 314a of the wall 314 with Kapton tape, and the region was prevented from being irradiated with corona. The water contact angle of a surface-treated region was 30°, and the water contact angle of the end surface 314a was kept at 85°.

An adhesive agent was used to attach the substrate 310 and the film to each other. The kind of the adhesive agent and the conditions of screen printing were similar to those in Example 1.

The adhesive agent applied to a surface of the substrate 310 was layered with the film and irradiated with ultraviolet light having a wavelength of 365 nm for 10 to 20 seconds using a UV-LED light source to initiate a curing reaction of the adhesive agent and bond the film onto the substrate 310.

### <Microchip Evaluation 2>

In the inlet 312, a pressure required for peeling the end surface 314a and the adhesive agent was measured by a method similar to that in Example 1 using a silicon tube and a connector produced by a bush made of an ABS resin. A pressure was applied from a through-hole closer to a storage portion 322. As a result of the experiment, liquid leakage occurred at points except the wall 314 at 800 kPa in a produced microchip in which the substrate 310 and the film were untreated. In contrast, in a microchip produced after surface treatment of an area other than a region except the end surface of substrate 310 and a region corresponding to the end surface of the film, the adhesive agent on the end surface 314a of the wall 314 was peeled at 600 kPa, and distilled water in the storage portion 322 flowed into the following flow path.

A similar experiment was also conducted in a microchip produced by corona treatment of the entire surface without limitation of a region in which surface treatment of the film was performed. As a result, an adhesive agent on the end surface 314a of the wall 314 was peeled at 600 kPa, and distilled water in the storage portion 322 flowed into the following flow path. This revealed that it was not necessary to untreat a region corresponding to the end surface 314a closer to the film.

The above results revealed that a liquid was able to be passed at optional timing by pressurization by keeping only an untreated end surface of the wall of a substrate, and surface-treating the other region to control the degree of bonding strength.

### EXAMPLE 3

Examination for decreasing a pressure required for peeling an adhesive agent and an end surface of a wall. By decreasing the required pressure, a liquid flow rate in passage of the liquid can be reduced, and an unexpected problem such as leakage of a reagent or the like sealed in a flow path in advance can be avoided. Since a liquid can be passed with manual pressure or a simple member such as an assembly-type blister pouch by decreasing a pressure required for passing a liquid, it is not necessary to use a peripheral device such as a pump or a syringe.

### <Microchip Preparation 3>

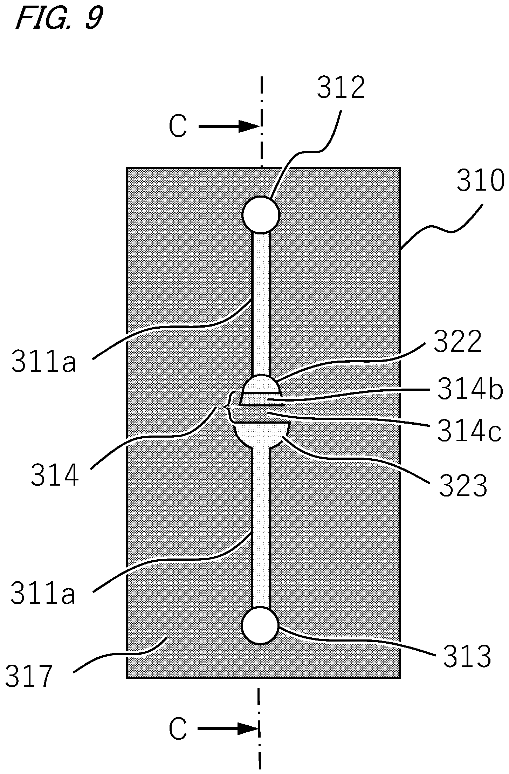

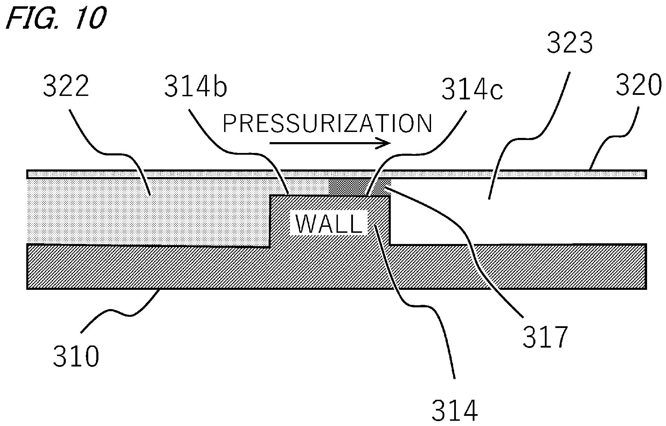

Production of a microchip according to Example 3 is described with reference to Figs. 9 and 10. A substrate 310 similar to the substrate in Example 1 was used as a substrate used in the production. ZF14-050 was used as a film 320. In the substrate 310, a region other than an end surface (end surface 314b and end surface 314c) of a wall 314 was subjected to atmospheric pressure plasma treatment. The entire surface of a film 320 was surface-treated.

A groove 311a and a storage portion 322 having a semicircular shape formed by cutting a circle having a diameter of 2 mm were filled with distilled water by dipping a through-hole (inlet 312) into distilled water under reduced pressure.

An adhesive agent 317 was used to attach the substrate 310 and the film 320 to each other. The adhesive agent 317 was TB3094, a solvent-free, radical reactive acrylic UV curing adhesive agent. The adhesive agent 317 was applied to a surface provided with the groove 311a of the substrate 310 by screen printing. In such a case, a case in which the adhesive agent 317 was not applied to around the half (end surface 314b), closer to the storage portion 322, of the end surface of the wall 314, and, in contrast, a case in which the adhesive agent 317 was not applied to around the half (end surface 314c), closer to a storage portion 323, of the end surface were prepared, as illustrated in Figs. 9 and 10. In a screen plate used, the mesh count was 640, the opening ratio was 39%, and the application thickness of the adhesive agent 317 was about 7 µm. Fig. 9 illustrates an application portion of the adhesive agent 317 of the substrate 310, and the end surface 314b closer to the storage portion 322 illustrates a state in which the adhesive agent 317 is not applied. Fig. 10 is a diagram illustrating a portion of a cross section, taken along the line C-C, of the microchip in which the film 320 is attached to the substrate 310 illustrated in Fig. 9.

The adhesive agent applied surface of the substrate 310 was layered with the film 320 and irradiated with ultraviolet light having a wavelength of 365 nm for 10 to 20 seconds using a UV-LED light source to initiate a curing reaction of the adhesive agent 317 and bond the film 320 onto the substrate 310.

### <Microchip Evaluation 3>

In the inlet 312, a pressure required for passing a liquid was measured by a method similar to that in Example 1 using a silicon tube and a connector produced by a bush made of an ABS resin. A pressure was applied from a through-hole (inlet 312) closer to the storage portion 322. As a result of the experiment, in the microchip in which the adhesive agent 317 was not applied to the end surface 314b, closer to the storage portion 322, of the end surface of the wall 314, the adhesive agent 317 on the end surface 314c of the wall 314 was peeled at 50 kPa, and distilled water in the storage portion 322 flowed into the following flow path. In contrast, in the microchip in which the adhesive agent 317 was not applied to the end surface 314c, closer to the storage portion 323, of the end surface of the wall 314, 500 kPa was required for passing a liquid.

It was found that a liquid was able to be passed at a lower pressure in a produced microchip in which the adhesive agent 317 was not applied to the flow path side (end surface 314b) to which the pressure of the end surface of the wall 314 was applied. An effect can also be obtained by reducing the volume of a pneumatic source that applies a pressure from the viewpoint of reducing the flow rate of a liquid in passage of the liquid. Since the volume of gas is inversely proportional to a pressure, the amount of compressed air required for passing a liquid can be reduced by reducing the size of the pneumatic source. As a result, leakage of a liquid at an unexpected point due to instantaneous returning of the volume of air compressed in passage of a liquid to an original volume can be prevented.

### DESCRIPTION OF REFERENCE SIGNS

100 ··· Microchip, 110 ··· Substrate, 111 ··· Flow path, 111a ··· Groove 112 ··· Inlet, 113 ··· Outlet, 114 ··· Wall, 114a ··· End surface, 115 ··· Bonding surface, 116 ··· Storage portion, 117a ··· Adhesive agent, 117b ··· Adhesive agent, 120 ··· Film, 200 ··· Microchip, 211 ··· Flow path, 214 ··· Wall, 216 ··· Washing liquid tank, 217b ··· Adhesive agent, 221 ··· Inlet, 222 ··· Storing portion, 223 ··· Reaction field, 224 ··· Stirring bar, 225 ··· Waste liquid tank, 226 ··· Flow path, 310 ··· Substrate, 311a ··· Groove, 312 ··· Inlet, 313 ··· Outlet, 314 ··· Wall, 314a, 314b, 314c ··· End surface, 317 ··· Adhesive agent, 320 ··· Film, 322 ··· Storage portion having semicircular shape formed by half-cutting circle having diameter of 2 mm, 323 ··· Storage portion having semicircular shape formed by half-cutting circle having diameter of 4 mm

## Claims

1. A microchip for analyzing a liquid sample, internally comprising a flow path, the microchip comprising:
a substrate comprising a bonding surface provided with a groove that forms the flow path, a storage portion that stores a liquid in a portion of the groove, and a wall that blocks flow of the liquid from the storage portion to the flow path;
a film that forms the flow path by attaching the film to the bonding surface of the substrate and covering the groove; and
an adhesive portion that is formed by an adhesive agent for adhesively bonding the bonding surface of the substrate and the film to each other by applying the adhesive agent to a region, excluding the groove and the storage portion, of the bonding surface, and an adhesive agent that is applied between an end surface of the wall and the film, and allows the liquid to be passed by peeling the end surface of the wall and the film by pressurization to the liquid.

2. The microchip according to claim 1, wherein
the substrate or the film comprises a through-hole serving as an inlet for pressurizing the liquid and an outlet for flow of the liquid or air at positions of both ends of the flow path formed by attaching the substrate and the film to each other.

3. The microchip according to claim 2, wherein
the liquid stored in the storage portion is pressurized using a micro syringe or a pump, or pressurized by press with a finger, through the inlet.

4. The microchip according to any one of claims 1 to 3, wherein
a width of the flow path is less than a width of the wall.

5. The microchip according to any one of claims 1 to 4, wherein
the end surface of the wall is subjected to surface treatment that reduces adhesive strength of the adhesive agent.

6. The microchip according to any one of claims 1 to 5, wherein
the liquid is any of the liquid sample, liquid comprising a reactant that is allowed to react with the liquid sample, and a washing liquid.

7. The microchip according to any one of claims 1 to 6, wherein
the substrate is any of plastic, silicone, and glass.

8. The microchip according to any one of claims 1 to 7, wherein
a region, excluding the groove, the storage portion, and the end surface of the wall, of the bonding surfaces, is subjected to hydrophilization treatment in the substrate.

9. The microchip according to any one of claims 1 to 8, wherein
the adhesive agent is a UV curing adhesive agent.

10. The microchip according to any one of claims 1 to 9, wherein
the adhesive agent is applied to the substrate by screen printing.

11. The microchip according to claim 10, wherein
the adhesive agent applied by the screen printing has a film thickness of 5 to 15 µm.

12. The microchip according to claim 10 or 11, wherein
the adhesive agent applied by the screen printing has a viscosity of 2,000 to 31,000 mPa s.

13. The microchip according to any one of claims 1 to 12, wherein
the film is cycloolefin polymer (COP), cycloolefin copolymer (COC), polymethyl methacrylate (PMMA), polystyrene (PS), polycarbonate (PC), or polyethylene terephthalate (PET).

14. The microchip according to any one of claims 1 to 13, wherein
the film has a thickness of 50 to 200 µm.

15. The microchip according to any one of claims 1 to 14, wherein
the adhesive agent between the end surface of the wall and the film is applied to a downstream portion of the flow path of the liquid in the end surface of the wall.

16. A method of producing a microchip for analyzing a liquid sample, internally comprising a flow path, the method comprising:
a step of preparing a substrate comprising a bonding surface provided with a groove that forms the flow path, a storage portion that stores a liquid in a portion of the groove, and a wall that blocks flow of the liquid from the storage portion to the flow path;
a step of preparing a film that forms the flow path by attaching the film to the bonding surface of the substrate and covering the groove;
a step of applying an adhesive agent to a region, excluding the groove and the storage portion, of the bonding surface, the adhesive agent adhesively bonding the bonding surface of the substrate and the film to each other and allowing the liquid to be passed by peeling an end surface of the wall and the film by pressurization to the liquid; and
a step of attaching the film to the bonding surface of the substrate.
